# EUROPEAN PATENT SPECIFICATION

(11) **EP 4 574 085 B1**
(45) Date of publication and mention of the grant of the patent: **03.06.2026**
(21) Application number: 24219110.4
(22) Date of filing: 11.12.2024
(51) Int. Cl.: A61B 90/70, A61L 2/26, A61B 50/30, E06B 7/23, F16J 15/00, F16J 15/3232, F16J 13/02

(54) **HERMETIC SEALING ASSEMBLY**
HERMETISCHE ABDICHTUNGSVORRICHTUNG
ENSEMBLE D'ÉTANCHÉITÉ HERMÉTIQUE

(30) Priority: 19.12.2023 IT 202300027099
(43) Date of publication of application: 25.06.2025
(73) Proprietor: Icos Pharma S.p.A., 33080 Zoppola (IT)
(72) Inventor: Beni, Stefano, 33170 Pordenone (PN) (IT); Bertolini, Gianni, 33078 San Vito al Tagliamento (PN) (IT)
(74) Representative: Petraz, Gilberto Luigi

(56) References cited:
- EP-A1- 2 915 946
- EP-A2- 1 700 070

## Description

### FIELD OF THE INVENTION

The present invention concerns a hermetic sealing assembly, preferably with double gasket, attached to a dividing wall and having an interface function between a reception zone for receiving instruments to be treated, for example surgical or laboratory instruments, and a sterile zone which comprises, for example, a treatment chamber of a sterilization machine.

### BACKGROUND OF THE INVENTION

For different laboratory and pharmaceutical applications, it is necessary to isolate and contain possible hazardous contaminating agents in an enclosed environment, in order to guarantee an adequate biosafety level (BSL - BioSafety Level). The biosafety level ranges from BSL-1, that is, the least hazardous level 1, to BSL-4, that is, the most hazardous level 4. Prior art assmeblies are disclosed in EP 2 915 946 A1 and EP 1 700 070 A2.

For this purpose, hermetic sealing assemblies attached to a dividing wall and having an interface function between a reception zone for receiving instruments to be treated, which may already have been pre-treated but are still contaminated, and a sterile zone are known. In the sterile zone there is usually a treatment chamber of a machine for sterilizing the instruments, such as an autoclave, which is hermetically associated with the sealing assembly.

The sealing assembly comprises a structure defined by a support frame provided with a first aperture and perimetrically and hermetically associated with the dividing wall, and by a central rim perimetrically and hermetically associated with the first aperture and provided with a second aperture hermetically connected to the treatment chamber.

To guarantee the hermetic seal between the support frame and the central rim, two flexible bio-containment gaskets are attached in correspondence with respective perimeter edges, which are reciprocally facing. These perimeter edges are conformed so as to have a U-like shape inclined by 90° that provides a rest surface for the two gaskets.

The two gaskets are disposed in series, and a closed isolation chamber is created between them to prevent the transfer of contaminating agents. In particular, one of the two gaskets is disposed on the side of the reception zone, and the other on the side of the sterile zone.

Bolts are used to attach the gaskets to the perimeter edges, which close the gaskets pack-wise and have a head end facing the reception zone and a terminal end where the clamping nut is located, the terminal end facing the sterile zone.

A disadvantage of such sealing assemblies is that the bolts pass from one side of the assembly to the other and, in the event that there is not a perfect seal or sealing, in correspondence with the terminal end of the bolt, contaminating agents can pass from the reception zone to the sterile zone. This may result in other disadvantages related to the dispersion of such contaminating agents into zones that are not hermetically sealed.

There is therefore the need to perfect a hermetic sealing assembly that can overcome at least one of the disadvantages of the state of the art.

To do this, it is necessary to solve the technical problem of providing a hermetic sealing assembly that prevents the possible passage of contaminating agents toward the sterile zone, even if there is not a perfect seal between the terminal end of the bolts and the gasket.

One purpose of the present invention is to provide a hermetic sealing assembly that is simple and economical to produce.

Another purpose of the present invention is to provide a hermetic sealing assembly that is effective and that guarantees the hermetic seal while respecting a high level of biosafety.

The Applicant has devised, tested and embodied the present invention to overcome the shortcomings of the state of the art and to obtain these and other purposes and advantages.

### SUMMARY OF THE INVENTION

The present invention is set forth and characterized in the independent claim.

The dependent claims describe other characteristics of the present invention or variants to the main inventive idea.

In accordance with the above purposes and to resolve the technical problem described above in a new and original way, also achieving considerable advantages compared to the state of the prior art, a hermetic sealing assembly according to the present invention comprises both a support frame provided with a first aperture delimited by first perimeter edges and also a central rim perimetrically and hermetically associated with the first perimeter edges.

The support frame is able to be perimetrically and hermetically attached in correspondence with an aperture of a dividing wall, which separates a reception zone for receiving contaminated instruments to be treated from a sterile zone which comprises a treatment chamber for the instruments. The instruments can be surgical or laboratory instruments.

The central rim is provided with second perimeter edges facing the first edges of the support frame, and with a second aperture hermetically connectable to the treatment chamber.

At least two sealing gaskets are attached between the first and second edges, disposed in series and engaged pack-wise by attachment elements.

In accordance with one aspect of the present invention, the first and second edges have shaped profiles, each comprising at least two consecutive undercuts protruding toward the reception zone and defining respective abutment portions for corresponding external and internal perimeter zones of the gaskets, which are clamped from one side to the other by the attachment elements so that corresponding head and terminal ends of the latter remain in the reception zone.

Doing so achieves the advantage that even if there is not a perfect seal between the gaskets and the head or terminal ends of the clamping elements, any contaminating agents are not dispersed in the sterile zone, but remain in the reception zone.

The at least two undercuts are spaced apart from each other, defining a hollow space in which the gaskets are disposed, and created in such a way as to converge from the reception zone toward the sterile zone so that the hollow space has a greater width toward the reception zone and a smaller width toward the sterile zone.

The at least two undercuts define, in the reception zone, on opposite sides with respect to the hollow space, respective collection compartments for collecting possible leakages of contaminating agents from the terminal ends, which can be engaged by a clamping element which stabilizes the attachment of the gaskets.

The at least two undercuts are conformed in such a way as to form a stepped profile, in which a rest part of each step defines a corresponding abutment portion.

In particular, the abutment portions of the undercuts of the first edges are aligned with corresponding abutment portions of the undercuts of the second edges, thus forming respective positioning planes, parallel and spaced apart from each other, for the gaskets.

The at least two gaskets have a rim-like shape, wherein a first gasket has larger perimeter sizes than a second gasket facing the sterile zone.

The sealing assembly can comprise a plurality of tightening rods which have a U-shaped cross section and a rest base. In particular, the tightening rods are associated with the gaskets so that the respective base is interposed between the head ends of the clamping elements and the corresponding external and internal perimeter zones of the gaskets.

Advantageously, the conformation of the first and second edges, and the disposition of the gaskets allows to guarantee a biosafety level 4 (BSL-4).

According to some embodiments, the profiles of the first and second edges each comprise three or more consecutive undercuts, to allow the disposition of as many sealing gaskets clamped from one side to the other by the attachment elements, so that corresponding head and terminal ends of the latter remain in the reception zone.

The present invention also concerns a dividing wall comprising a sealing assembly as disclosed above having an interface function between a reception zone for receiving contaminated instruments to be treated and a sterile zone comprising a treatment chamber of a machine for sterilizing the instruments.

### DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a partly sectioned top view of a hermetic sealing assembly according to the present invention, which is hermetically attached to a dividing wall that separates a reception zone for receiving contaminated instruments to be treated from a sterile zone in which there is a treatment chamber;
- fig. 2 is an enlarged detail of the sealing assembly of fig. 1;

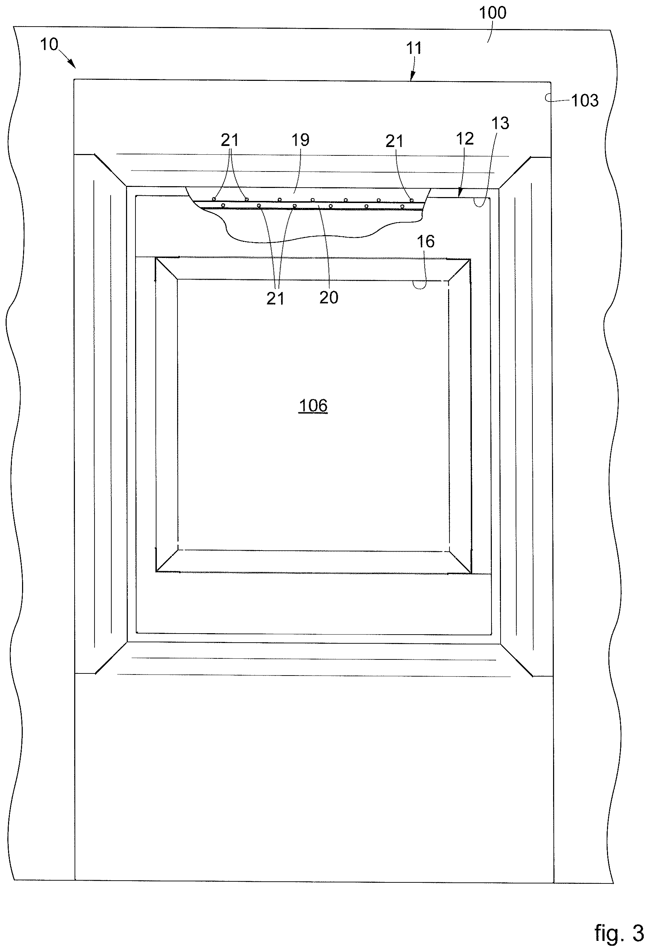

- fig. 3 is a front view of the hermetic sealing assembly of fig. 1 attached to the dividing wall.

We must clarify that the phraseology and terminology used in the present description, as well as the figures in the attached drawings also in relation as to how described, have the sole function of better illustrating and explaining the present invention, their purpose being to provide a non-limiting example of the invention itself, since the scope of protection is defined by the claims.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF SOME EMBODIMENTS OF THE PRESENT INVENTION

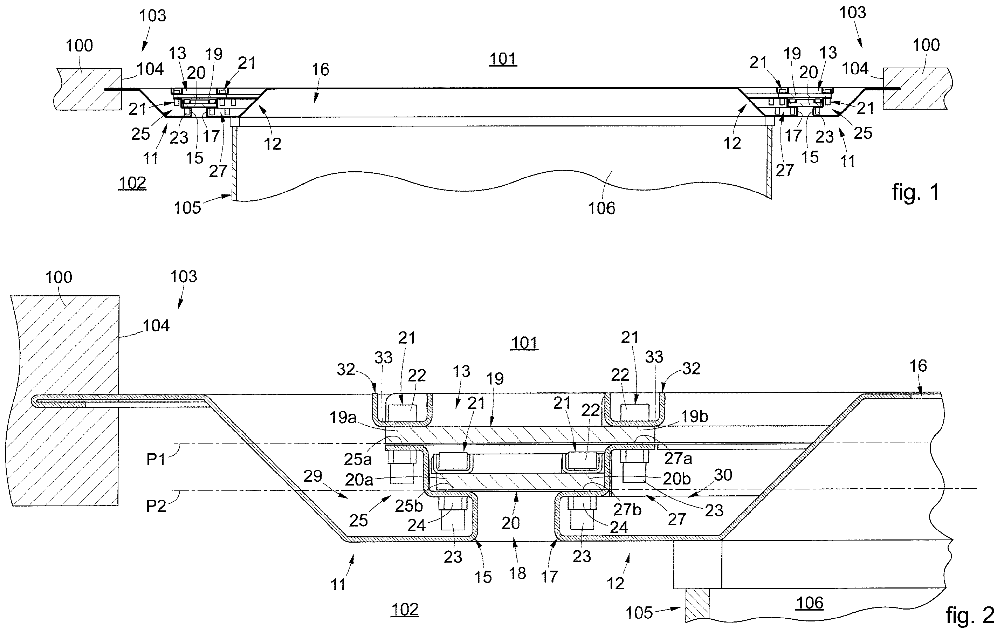

With reference to fig. 1, a hermetic sealing assembly 10 according to the present invention is suitable and usable in a dividing wall 100 and has an interface function between a reception zone 101 for receiving contaminated instruments to be treated, and a sterile zone 102.

The instruments can be surgical, or laboratory instruments used for operations or experiments.

In the reception zone 101, the instruments may have already been pre-treated, for example by means of washing and/or thermo-disinfection, but they are still partly contaminated. For example, the instruments may be contaminated with hazardous biological agents that have to be contained in closed and hermetically sealed environments.

The sterile zone 102 comprises a treatment chamber 106 in which the instruments are treated, in particular sterilized and/or sanitized.

The treatment chamber 106 can be part of a sterilization machine 105. For example, the sterilization machine 105 can be an autoclave comprising the treatment chamber 106 in which the contaminated instruments, possibly pre-treated, are sterilized using high temperature steam.

The dividing wall 100 is provided with a main aperture 103 delimited by main edges 104.

The sealing assembly 10 comprises a support frame 11 perimetrically and hermetically attached to the main edges 104. The support frame 11 is provided with a first central aperture 13 delimited by first perimeter edges 15.

The sealing assembly 10 also comprises a central rim 12 perimetrically and hermetically associated with the first perimeter edges 15 and provided with second perimeter edges 17 facing the latter. The central rim 12 comprises a second central aperture 16 which is hermetically connected to the treatment chamber 106 to allow the introduction of the contaminated instruments.

It should be noted that the treatment chamber 106 can be provided at entry with suitable hermetic closure means, not shown in the drawings, configured to selectively close the treatment chamber 106 itself with respect to the reception zone 101.

The first 15 and second 17 edges are reciprocally facing and spaced apart from each other, so that a hollow space 18 is formed between them.

Two sealing gaskets 19, 20 are attached between the first 15 and second 17 edges, also called bio-containment gaskets, which are disposed in series in the hollow space 18 and engaged pack-wise by corresponding attachment elements 21 cooperating with the corresponding first 15 and second 17 edges.

A closed isolation chamber is created between the two gaskets 19, 20, which prevents the transfer of contaminating agents from the reception zone 101 to the sterile zone 102.

Each attachment element 21 comprises a head, or proximal, end 22 and a terminal, or distal, end 23.

The terminal end 23 can cooperate with a clamping element 24 that stabilizes the attachment of the gaskets 19, 20.

The attachment element 21 can be a bolt and the clamping element 24 can be a nut. It is clear that other suitable attachment means can be used to attach the gaskets 19, 20 to the first 15 and second 17 edges.

Preferably, there is a first 19 and a second 20 gasket, wherein the second gasket 20 faces the sterile zone 102.

The gaskets 19, 20 have a rim-like shape and comprise respective external perimeter zones 19a, 20a attached to the first edges 15 and internal perimeter regions 19b, 20b attached to the second edges 17.

The first 15 and second 17 edges have respective profiles each comprising at least two consecutive undercuts 25 and 27, protruding toward the reception zone 102 and defining respective abutment portions 25a, 25b, 27a, 27b for the corresponding external 19a, 20a and internal 19b, 20b perimeter zones, which are clamped from one side to the other by the attachment elements 21 so that corresponding head 22 and terminal 23 ends of the latter remain in the reception zone 101.

This offers the advantage that even if there is not a perfect seal between the gaskets 19, 20 and the head 22 or terminal 23 ends of the clamping elements 21, any contaminating agents are not dispersed in the sterile zone 102, but remain in the reception zone 101. Another advantage is that open and non-blind nuts can also be used as clamping elements 24.

The two undercuts 25 and 27 are made so as to converge from the reception zone 101 toward the sterile zone 102. Therefore, the hollow space 18 has a greater width on the side of the reception zone 101 and a smaller width on the side of the sterile zone 102.

The undercuts 25 and 27 define, in the reception zone 101, on opposite sides with respect to the hollow space 18, respective collection compartments 29 and 30 that allow to contain any leakages of contaminating agents from the terminal ends 23 of the attachment elements 21.

The two undercuts 25 and 27 are conformed so as to form a stepped profile, in which a rest part of each step defines a corresponding abutment portion 25a, 25b, 27a, 27b. We must clarify that the rest part is parallel to the dividing wall 100.

The abutment portions 25a, 25b of the undercuts 25 of the first edges 15 are aligned with corresponding abutment portions 27a, 27b of the undercuts 27 of the second edges 17, so as to form respective positioning planes P1, P2, parallel and spaced apart from each other, of the gaskets 19, 20.

The first gasket 19 is positioned on a first positioning plane P1 and has larger perimeter sizes than the second gasket 20, which is positioned on a second positioning plane P2.

The sealing assembly 10 comprises a plurality of tightening rods 32 which have a U-shaped cross section and a rest base 33. The tightening rods 32 are associated with the gaskets 19, 20 so that the respective base 33 is interposed between the head end 22 of each attachment element 21 and the corresponding external 19a, 20a and internal 19b, 20b perimeter zone of the gaskets 19, 20.

The first 15 and second 17 edges can have profiles each comprising three or more consecutive undercuts 25, 27, as defined above, to allow the disposition of as many sealing gaskets, which are clamped from one side to the other by the attachment elements 21 so that the corresponding head 22 and terminal 23 ends of the latter remain in the reception zone 101.

The conformation of the first 15 and second 17 perimeter edges and the disposition of the gaskets 19, 20 allow the sealing assembly 10 to guarantee a biosafety level 4 (BSL-4).

It is clear that modifications and/or additions of parts or steps may be made to the sealing assembly 10 as described heretofore, without thereby departing from the field and scope of the present invention, as defined by the claims.

## Claims

1. Hermetic sealing assembly (10) comprising both a support frame (11) provided with a first aperture (13) delimited by first perimeter edges (15) and able to be perimetrically and hermetically attached to a dividing wall (100) which separates a reception zone (101) for receiving contaminated instruments to be treated from a sterile zone (102), and also a central rim (12) provided with second edges (17) facing said first perimeter edges (15) with which it is associated perimetrically and hermetically, and with a second aperture (16) hermetically connectable to a treatment chamber (105) of said sterile zone (102), wherein between said first (15) and second (17) edges at least two sealing gaskets (19, 20) are attached, disposed in series and engaged pack-wise by attachment elements (21), wherein said first (15) and second (17) edges have profiles each comprising at least two consecutive undercuts (25, 27) protruding toward said reception zone (101) and defining respective abutment portions (25a, 25b, 27a, 27b) for corresponding external (19a, 20a) and internal (19b, 20b) perimeter zones of said gaskets (19, 20) which are clamped from one side to the other by said attachment elements (21) so that corresponding head (22) and terminal (23) ends of the latter remain in said reception zone (101).

2. Sealing assembly (10) as in claim 1, **characterized in that** said at least two undercuts (25, 27) of said first (15) and second (17) edges are spaced apart from each other, defining a hollow space (18) in which said gaskets (19, 20) are disposed, and created in such a way as to converge from said reception zone (101) toward said sterile zone (102) so that said hollow space (18) has a greater width toward the reception zone (101) and a smaller width toward the sterile zone (102).

3. Sealing assembly (10) as in claim 2, **characterized in that** said at least two undercuts (25, 27) define, in said reception zone (101), on opposite sides with respect to said hollow space (18), respective collection compartments (29, 30) for collecting possible leakages of contaminating agents from said terminal ends (23), which are engaged by a clamping element (24) which stabilizes the attachment of said gaskets (19, 20).

4. Sealing assembly (10) as in claim 1, 2 or 3, **characterized in that** said at least two undercuts (25, 27) are conformed in such a way as to form a stepped profile, in which a rest part of each step defines a corresponding abutment portion (25a, 25b, 27a, 27b).

5. Sealing assembly (10) as in any claim hereinbefore, **characterized in that** abutment portions (25a, 25b) of the undercuts (25) of said first edges (15) are aligned with corresponding abutment portions (27a, 27b) of the undercuts (27) of said second edges (17), thus forming respective positioning planes (P1, P2), parallel and spaced apart from each other, for said gaskets (19, 20).

6. Sealing assembly (10) as in any claim hereinbefore, **characterized in that** said at least two gaskets (19, 20) have a rim-like shape, wherein a first gasket (19) has larger perimeter sizes than a second gasket (20) facing said sterile zone (102).

7. Sealing assembly (10) as in any claim hereinbefore, **characterized in that** it comprises a plurality of tightening rods (32) which have a U-shaped cross section and a rest base (33), and which are associated with said gaskets (19, 20) so that the respective base (33) is interposed between said head ends (22) of said clamping elements (21) and said corresponding external (19a, 20a) and internal (19b, 20b) perimeter zones.

8. Sealing assembly (10) as in any claim hereinbefore, **characterized in that** the conformation of said first (15) and second (17) edges and the disposition of said gaskets (19, 20) allows to guarantee a Biosafety level 4 (BSL-4).

9. Sealing assembly (10) as in any claim hereinbefore, **characterized in that** said first (15) and second (17) edges have profiles each comprising three or more consecutive undercuts (25, 27), to allow the disposition of as many sealing gaskets clamped from one side to the other by said attachment elements (21) so that corresponding head (22) and terminal (23) ends of the latter remain in said reception zone (101).

10. Dividing wall (100) comprising a sealing assembly (10) as in any claim hereinbefore having an interface function between a reception zone (101) for receiving contaminated instruments to be treated and a sterile zone (102) comprising a treatment chamber (106) of a machine (105) for sterilizing said instruments.

## Patentansprüche

1. Hermetische Dichtungsanordnung (10), aufweisend sowohl einen Halterahmen (11), der mit einer ersten Öffnung (13) versehen ist, die durch erste Umfangsränder (15) begrenzt ist, und der umfänglich hermetisch an einer Trennwand (100) befestigt werden kann, welche einen Aufnahmebereich (101) zur Aufnahme von zu behandelnden kontaminierten Instrumenten von einem sterilen Bereich (102) trennt, als auch einen zentralen Kranz (12), der mit zweiten Rändern (17), die den ersten Umfangsrändern (15) zugewandt sind und mit denen er umfänglich hermetisch verbunden ist, sowie mit einer zweiten Öffnung (16) versehen ist, die mit einer Behandlungskammer (105) des sterilen Bereichs (102) hermetisch verbunden werden kann, wobei zwischen den ersten (15) und den zweiten (17) Rändern mindestens zwei Dichtungen (19, 20) befestigt sind, die in Reihe angeordnet sind und mit Befestigungselementen (21) packweise in Eingriff stehen, wobei die ersten (15) und die zweiten (17) Ränder Profile haben, die jeweils mindestens zwei aufeinanderfolgende Hinterschneidungen (25, 27) aufweisen, die in Richtung zum Aufnahmebereich (101) vorstehen und jeweilige Anlageabschnitte (25a, 25b, 27a, 27b) für korrespondierende äußere (19a, 20a) und innere (19b, 20b) Umfangsbereiche der Dichtungen (19, 20) definieren, die mittels der Befestigungselemente (21) von einer Seite zur anderen geklemmt werden, so dass korrespondierende Kopf- (22) und Abschluss (23)-Enden der Letzteren im Aufnahmebereich (101) verbleiben.

2. Dichtungsanordnung (10) nach Anspruch 1, **dadurch gekennzeichnet, dass** die mindestens zwei Hinterschneidungen (25, 27) der ersten (15) und der zweiten (17) Ränder voneinander im Abstand sind, definierend einen Hohlraum (18), in dem die Dichtungen (19, 20) angeordnet sind, und so ausgebildet, dass sie vom Aufnahmebereich (101) aus zum sterilen Bereich (102) hin konvergieren, sodass der Hohlraum (18) zum Aufnahmebereich (101) hin eine größere Breite und zum sterilen Bereich (102) hin eine kleinere Breite hat.

3. Dichtungsanordnung (10) nach Anspruch 2, **dadurch gekennzeichnet, dass** die mindestens zwei Hinterschneidungen (25, 27) in dem Aufnahmebereich (101) in Bezug auf den Hohlraum (18) auf entgegengesetzten Seiten jeweilige Sammelkammern (29, 30) zum Sammeln möglicher Leckagen von Kontaminationsstoffen aus den Abschluss-Enden (23) bilden, die mit einem Klemmelement (24) in Eingriff sind, welches die Befestigung der Dichtungen (19, 20) stabilisiert.

4. Dichtungsanordnung (10) nach Anspruch 1, 2 oder 3, **dadurch gekennzeichnet, dass** die mindestens zwei Hinterschneidungen (25, 27) so ausgebildet sind, dass sie ein abgestuftes Profil bilden, wobei ein Auflageabschnitt jeder Stufe einen korrespondierenden Anlageabschnitt (25a, 25b, 27a, 27b) definiert.

5. Dichtungsanordnung (10) nach irgendeinem vorstehenden Anspruch, **dadurch gekennzeichnet, dass** die Anlageabschnitte (25a, 25b) der Hinterschneidungen (25) der ersten Ränder (15) mit korrespondierenden Anlageabschnitten (27a, 27b) der Hinterschneidungen (27) der zweiten Ränder (17) ausgerichtet sind und so für die Dichtungen (19, 20) jeweilige Positionierungsebenen (P1, P2) bilden, die parallel zueinander und voneinander im Abstand sind.

6. Dichtungsanordnung (10) nach irgendeinem vorstehenden Anspruch, **dadurch gekennzeichnet, dass** die mindestens zwei Dichtungen (19, 20) eine kranzartige Form haben, wobei eine erste Dichtung (19) größere Umfangsabmessungen hat als eine zweite Dichtung (20), die dem sterilen Bereich (102) zugewandt ist.

7. Dichtungsanordnung (10) nach irgendeinem vorstehenden Anspruch, **dadurch gekennzeichnet, dass** sie eine Vielzahl von Spannstangen (32) aufweist, die einen U-förmigen Querschnitt und eine Auflagebasis (33) haben, und die den Dichtungen (19, 20) zugeordnet sind, so dass die jeweilige Auflagebasis (33) zwischen den Kopf-Enden (22) der Klemmelemente (21) und den korrespondierenden äußeren (19a, 20a) und inneren (19b, 20b) Umfangsbereichen angeordnet ist.

8. Dichtungsanordnung (10) nach irgendeinem vorstehenden Anspruch, **dadurch gekennzeichnet, dass** die Formgebung der ersten (15) und der zweiten (17) Ränder sowie die Anordnung der Dichtungen (19, 20) es ermöglicht, ein Biosicherheitslevel 4 (BSL-4) zu gewährleisten.

9. Dichtungsanordnung (10) nach irgendeinem vorstehenden Anspruch, **dadurch gekennzeichnet, dass** die ersten (15) und die zweiten (17) Ränder Profile haben, die jeweils drei oder mehr aufeinanderfolgende Hinterschneidungen (25, 27) aufweisen, um die Anordnung von ebenso vielen Dichtungen zu ermöglichen, die von einer Seite zur anderen mittels der Befestigungselemente (21) geklemmt werden, so dass die korrespondierenden Kopf- (22) und Abschluss (23)-Enden der Letzteren in dem Aufnahmebereich (101) verbleiben.

10. Trennwand (100) mit einer Dichtungsanordnung (10) nach irgendeinem vorstehenden Anspruch, die eine Schnittstellenfunktion zwischen einem Aufnahmebereich (101) zum Aufnehmen von zu behandelnden kontaminierten Instrumenten und einem sterilen Bereich (102), der eine Behandlungskammer (106) einer Maschine (105) zum Sterilisieren der Instrumente aufweist, hat.

## Revendications

1. Ensemble d'étanchéité hermétique (10) comprenant à la fois un cadre de support (11) pourvu d'une première ouverture (13) délimitée par des premiers bords périmétriques (15) et apte à être fixé de manière périmétrique et hermétique à une paroi de séparation (100) qui sépare une zone de réception (101) destinée à recevoir des instruments contaminés à traiter d'une zone stérile (102), ainsi qu'un rebord central (12) pourvu de seconds bords (17) faisant face auxdits premiers bords périmétriques (15) auxquels il est associé de manière périmétrique et hermétique, et d'une seconde ouverture (16) apte à être connectée de manière hermétique à une chambre de traitement (105) de ladite zone stérile (102), lequel, entre lesdits premiers (15) et seconds (17) bords, au moins deux joints d'étanchéité (19, 20) sont fixés, disposés en série et serrés en paquet par des éléments de fixation (21), lequel lesdits premiers (15) et seconds (17) bords présentent des profils comprenant chacun au moins deux contre-dépouilles consécutives (25, 27) faisant saillie vers ladite zone de réception (101) et définissant respectivement des portions d'appui (25a, 25b, 27a, 27b) pour les zones périmétriques externe (19a, 20a) et interne (19b, 20b) correspondantes desdits joints (19, 20) qui sont serrés d'un côté à l'autre par lesdits éléments de fixation (21) de sorte que les extrémités de tête (22) et terminales (23) correspondantes de ces derniers restent dans ladite zone de réception (101).

2. Ensemble d'étanchéité (10) selon la revendication 1, **caractérisé en ce que** lesdites au moins deux contre-dépouilles (25, 27) desdits premiers (15) et seconds (17) bords sont espacées l'une de l'autre, définissant un espace creux (18) dans lequel sont disposés lesdits joints (19, 20), et réalisées de manière à converger depuis ladite zone de réception (101) vers ladite zone stérile (102) de sorte que ledit espace creux (18) présente une largeur plus grande du côté de la zone de réception (101) et une largeur plus petite du côté de la zone stérile (102).

3. Ensemble d'étanchéité (10) selon la revendication 2, **caractérisé en ce que** lesdites au moins deux contre-dépouilles (25, 27) définissent, dans ladite zone de réception (101), de part et d'autre par rapport audit espace creux (18), des compartiments de collecte respectifs (29, 30) pour la collecte d'éventuelles fuites d'agents contaminants provenant desdites extrémités terminales (23), lesquelles sont serrées par un élément de serrage (24) qui stabilise la fixation desdits joints (19, 20).

4. Ensemble d'étanchéité (10) selon l'une quelconque des revendications 1 à 3, **caractérisé en ce que** lesdites au moins deux contre-dépouilles (25, 27) sont conformées de manière à former un profil en escalier, dans lequel une partie d'appui de chaque marche définit une portion d'appui correspondante (25a, 25b, 27a, 27b).

5. Ensemble d'étanchéité (10) selon l'une quelconque des revendications précédentes, **caractérisé en ce que** les portions d'appui (25a, 25b) des contre-dépouilles (25) desdits premiers bords (15) sont alignées avec les portions d'appui correspondantes (27a, 27b) des contre-dépouilles (27) desdits seconds bords (17), formant ainsi des plans de positionnement respectifs (P1, P2), parallèles et espacés l'un de l'autre, pour lesdits joints (19, 20).

6. Ensemble d'étanchéité (10) selon l'une quelconque des revendications précédentes, **caractérisé en ce que** lesdits au moins deux joints (19, 20) présentent une forme de rebord, un premier joint (19) ayant des dimensions périmétriques plus grandes qu'un second joint (20) faisant face à ladite zone stérile (102).

7. Ensemble d'étanchéité (10) selon l'une quelconque des revendications précédentes, **caractérisé en ce qu'**il comprend une pluralité de tiges de serrage (32) qui présentent une section transversale en U et une base d'appui (33), et qui sont associées auxdits joints (19, 20) de sorte que la base respective (33) est interposée entre lesdites extrémités de tête (22) desdits éléments de serrage (21) et lesdites zones périmétriques externe (19a, 20a) et interne (19b, 20b) correspondantes.

8. Ensemble d'étanchéité (10) selon l'une quelconque des revendications précédentes, **caractérisé en ce que** la conformation desdits premiers (15) et seconds (17) bords et la disposition desdits joints (19, 20) permettent de garantir un niveau de biosécurité 4 (BSL-4).

9. Ensemble d'étanchéité (10) selon l'une quelconque des revendications précédentes, **caractérisé en ce que** lesdits premiers (15) et seconds (17) bords présentent des profils comprenant chacun trois contre-dépouilles consécutives ou plus (25, 27), afin de permettre la disposition d'autant de joints d'étanchéité serrés d'un côté à l'autre par lesdits éléments de fixation (21) de sorte que les extrémités de tête (22) et terminales (23) correspondantes de ces derniers restent dans ladite zone de réception (101).

10. Paroi de séparation (100) comprenant un ensemble d'étanchéité (10) selon l'une quelconque des revendications précédentes ayant une fonction d'interface entre une zone de réception (101) destinée à recevoir des instruments contaminés à traiter et une zone stérile (102) comprenant une chambre de traitement (106) d'une machine (105) de stérilisation desdits instruments.
